# EUROPEAN PATENT SPECIFICATION

(11) **EP 3 311 854 B1**
(45) Date of publication and mention of the grant of the patent: **05.08.2020**
(21) Application number: 17197245.8
(22) Date of filing: 19.10.2017
(51) Int. Cl.: A61L 27/34, A61L 27/50, A61L 27/54, A61L 27/58

(54) **A NANOCOMPOSITE LAYER ON THE BASIS OF COLLAGEN NANOFIBERS, AND A METHOD OF PREPARATION THEREOF**
NANOKOMPOSITSCHICHT AUF BASIS VON KOLLAGENNANOFASERN UND VERFAHREN ZUR HERSTELLUNG DAVON
COUCHE NANOCOMPOSITE À BASE DE NANOFIBRES DE COLLAGÈNE ET SON PROCÉDÉ DE PRÉPARATION

(30) Priority: 20.10.2016 CZ 20160656
(43) Date of publication of application: 25.04.2018
(73) Proprietor: Ústav Struktury A Mechaniky Hornin AV CR, V.V.I., 182 09 Praha 8 (CZ); Ceské Vysoké Ucení Technické V Praze Fakulta Strojni, 166 07 Praha 6 (CZ); ProSpon spol. s.r.o., 27201 Kladno (CZ); Contipro a.s., 56102 Dolni Dobrouc (CZ)
(72) Inventor: SUCHY, Tomas, 19000 Praha 9 (CZ); SUPOVA, Monika, 14900 Praha 11 (CZ); DENK, Frantisek, 15521 Praha 17 (CZ); RYGLOVA, Sarka, 10600 Praha 10 (CZ); ZALOUDKOVA, Margit, 25228 Cernosice (CZ); SUCHARDA, Zbynek, 10200 Praha 10 (CZ); BALLAY, Rastislav, 14300 Praha 4 (CZ); HORNY, Lukas, 53002 Pardubice (CZ); CEJKA, Zdenek, 27201 Kladno (CZ); POKORNY, Marek, 56164 Jablonne nad Orlici (CZ); KNOTKOVA, Katerina, 56501 Chocen (CZ); VELEBNY, Vladimir, 56401 Zamberk (CZ)
(74) Representative: Lunzarová, Lucie

(56) References cited:
- WO-A1-2015/162559
- WO-A2-2009/049565
- WO-A2-2009/131638
- TENG S H ET AL: "Collagen/hydroxyapatite composite nanofibers by electrospinning", MATERIALS LETTERS, ELSEVIER, AMSTERDAM, NL, vol. 62, no. 17-18, 30 June 2008 (2008-06-30), pages 3055-3058, XP022648772, ISSN: 0167-577X, DOI: 10.1016/J.MATLET.2008.01.104 [retrieved on 2008-02-07]

## Description

### Field of the Invention

The present invention relates to orthopaedic and dental implants based on metal or metal alloys coated with a bioactive collagen type I layer and hydroxyapatite, characterized in effective releasing of antibiotics. The bioactive submicron- and nanostructured layer is biodegradable, biocompatible, and/or bioresorbable. Said layer is prepared by the combination of electrospinning and electroblowing of the collagen solution comprising hydroxyapatite nanoparticles. The main subject of the present invention is the method of depositing antibiotics in order to achieve a more efficient releasing thereof without increasing the cytotoxicity. This method comprises impregnation of crosslinked sterilized collagen/hydroxyapatite layers with antibiotics in the form of a solution.

### Background of the Invention

With respect to substantial health risks and high costs associated with reoperations of current implants, namely big joints, the prolongation of their durability by functionalization of their surface is very perspective and desirable direction of development in the field of healthcare techniques. With regard to the fact that the number of such orthopaedic surgeries has significantly increased worldwide during the last decade, the implementation of modern types of implants with a bioactive surface, modified with the use of nanotechnology, thanks to their higher endurance and osteointegration, can lead both to a better patient's comfort, and to important costs savings. The metal implants based on titanium, titanium alloys, stainless steel, chrome and cobalt alloys, that have been the most commonly used so far, are biocompatible but bioinert. They are also often subject to various forms of corrosion in the body environment. The osteointegration of bioinert metal implants is complicated; their surface needs to be subsequently modified for an appropriate fixation with a bone. Physical and chemical or morphological modification of the surface of an endoprosthesis can be made to enhance the fixation. Nowadays, bioactive calcium phosphates, usually hydroxyapatite or tricalcium phosphate, are used for chemical modifications of metal implants. These bioceramical materials show excellent properties in combination with a bone thanks to their surface reactivity brought by the mechanism that forms a contact mineralized layer between the implant and the bone tissue. This interface ensures the physical and chemical and mechanical cohesion between the implant and the bone. However, the use of these materials in their original form is limited by their mechanical properties or adhesion with the implants' surface. A high content of hydroxyapatite results in an increase of fragility which can lead to a decrease of mechanical resistance and decrease of adhesion to the support. Another strategy can consist in coating with a polymer layer based on synthetic polymers (aliphatic polyesters and copolymers thereof). Bioactivity enhancement of the surface can be achieved by coating with natural polymers (collagen, gelatine). If these natural polymers in the form of nanofibers are supplemented with calcium phosphate in the form of nanoparticles, nanostructured surface ideal for appropriate adhesion and bone cells proliferation is achieved. Another advantage of these composite materials consists in the possibility of selection of the particular components according to their composition and orientation, material, physical, and chemical characteristics that can result in a wide range of mechanical and biological properties.

At the same time, these bioactive layers can serve as local carriers of antibiotics (ATB) for their gradual release as a prevention of osteomyelitis. Local application of antibiotics is more preferred as it enables a higher dosage; a short transport way and minimal ATB levels fluctuation in the blood stream also represent an advantage. Thus the antibiotics act also in avascular zones without increasing the systemic toxicity. Then, the antibiotic can affect both the residual planktonic bacteria and sessile forms in the biofilm. Furthermore, the local carriers of antibiotics fill the "dead space" developed after the removed necrotic tissues of the infected bone.

When non-biodegradable matrices useful as ATB carriers are concerned, polymethylmethacrylate is the most popular (often used as the bone cement). It has a drawback in a non-controlled, or even too fast release of ATB in too high concentrations and the necessity of another surgery after finishing the treatment (the treatment is two-step: healing, final implantation). Therefore, nowadays the effort is aimed at the research of biodegradable systems, for which it is supposed that no secondary surgery would be necessary. Furthermore, these systems provide the possibility of drug release control by means of physical and chemical configuration of the matrix/carriers and drugs. This system could provide for release of antibiotics in concentrations exceeding minimum inhibition concentrations for most of the known pathogens, without any adverse side effects. Gentamicin, vancomycin, and tobramycin, or combinations thereof are the most used antibiotics.

Controlled release rate depends on permeability, chemical modification of the matrix/carrier, layer thickness, crosslinking degree of the matrix/carrier, physical and chemical properties of the drug, interaction between the polymer matrix, the bound drug and the dissolution media, as well as on the arrangement of the components. The release of antibiotics in vitro is usually monitored by means of UV spectrometry or high-performance liquid chromatography (HPLC). Biological testing in vitro, performed on osteoblasts or mesenchymal stem cells, not only characterize the cytotoxicity of the material itself, but enable to directly assess the inhibitive potential of the concentration of the released antibiotic or the influence of the used methods of preparation (e.g. collagen crosslinking) on the biocompatibility of the studied material. Bacterial testing, characterizing the material antibacterial activity, is often performed on three bacterial stems, such as Gram-positive Staphylococci, Gram-negative Pseudomonades, and E. coli.

The antibiotics can be deposited by means of various methods. The basic method is simple mixing of the polymer and the antibiotic in the form of a powder or a solution. This method has the disadvantage of the use of organic solvents. Another conventional method is polymer soaking in the solution containing antibiotics. Nanofibrous layers enriched with antibiotics are often prepared by direct spinning from the polymer solution containing the said drug. Despite the fact that the conditions applied during the electrostatic spinning are very important for antibiotics stability, they are not taken into account. Some antibiotics, such as vancomycin, are thermosensitive and proven to transform into antibiotically inactive forms in a solution already at the room temperature within few hours. These inactive forms are not normally detected. The conventionally used UV/VIS spectrometry is not able to detect these antibiotically inactive forms and thus these analyses have to be performed with the use of liquid chromatography. Collagen nanolayers have to be further crosslinked after electrostatic spinning. During this procedure, they are washed out and the nanofibrous carrier becomes less effective, which, in the end, leads also to a low economical effectivity.

WO 2015/162559 describes a bioactive nanocomposite layer comprising collagen nanofibers with a diameter of 100 nm or less and comprising nanophased hydroxyapatite with a particle size of 50-1500 nm. The ratio of collagen to the calcium phosphate compound is 20:1 - 1:1. The collagen can be collagen type I, and the composition can be crosslinked, e.g. with EDC and NHS. The nanocomposite layer can be used as a coating on an orthopaedic or dental implant, and it can further comprise an active agent.

The aim of the invention is, i.a., to prepare a nanocomposite layer that imitates the composition and the structure of a real bone as well as possible, i.e. so that it contains only collagen and calcium phosphate/hydroxyapatite, which leads to better osteoinductive properties of the said surface layer of an implant. Thus, the presence of any additives (except antibiotics), including poly(ethylene oxide) (PEO) is undesirable. The use of PEO is preferable for achieving the effective spinning process but thanks to sufficient, almost ideal, washing of PEO, optimal predefined layer composition is assured.

### Summary of the Invention

The scope of this invention is defined by the claims. Embodiments in the description relating to methods of treatment are not covered by the claims. Any "embodiment" or "example" which is disclosed in the description but is not covered by the claims should be considered as presented for illustrative purpose only.

Drawbacks and disadvantages of the prior art are largely eliminated by the method of preparation of a bioactive nanocomposite layer based on collagen nanofibers for orthopaedic and dental implants containing nanofibers having the mean diameter value of 100 - 350 nm, defined by claim 1. The nanofibers comprise 75 to 100 weight % of collagen type I, preferably 85 to 100 weight %, wherein the said nanofibers comprise at most 1 weight % of poly(ethylene oxide). The aim is to achieve zero content of poly(ethylene oxide); however it is likely to be always present in trace amount. Thus the expression "at most 1 weight % of poly(ethylene oxide)" refers the poly(ethylene oxide) content in the range of 0 to 1 weight %.

Preferably, the nanocomposite layer comprises 5 to 15 weight % of antibiotic, preferably 7 to 12 weight %, preferably 9 to 11 weight %, wherein the antibiotic is selected from the group comprising vancomycin, gentamicin, and tobramycin, and the combinations thereof.

The nanocomposite layer further comprises 5 to 15 weight % of hydroxyapatite in the form of nanoparticles of the size of 50 - 250 nm.

The prior art drawbacks are largely eliminated by an orthopaedic or teeth implant based on metal or metal alloys that is at least partly coated with the above mentioned bioactive nanocomposite layer and that is intended for human or veterinary use.

The layer prepared according to the invention may be used for coating orthopaedic and dental implants based on metal or metal alloys.

The nanocomposite layer prepared according to the invention is made according to the following procedure: collagen type I and poly(ethylene oxide) having the viscosity average molecular weight in the range of 400,000 to 900,000, in the ratio of poly(ethylene oxide) to collagen of 0.05 to 0.11, are mixed with phosphate buffer for obtaining the concentration of 5 to 11 weight % of collagen in the mixture, the mixture is subjected to the temperature in the range of 32 to 42°C for at least 60 hours, then it is homogenized by mixing while concurrently adding ethanol and hydroxyapatite, with the final volume ratio of phosphate buffer to ethanol 1:1 to 1:2, then the mixture undergoes electrostatic spinning while forming the nanocomposite layer, that is subsequently washed with saline solution or phosphate buffer and then with water and is dried.

### Brief description of the drawings

The invention is further described in more detail by means of examples and drawings, where the drawings show as follows:
Fig. 1. Concentration of the active form of vancomycin (median, interquartile range) released from materials comprising 0 weight % (fig. 1a), 5 weight % (Fig. 1b), and 15 weight % (Fig. 1c) of hydroxyapatite prepared by three different methods.
Fig. 2. Size of inhibition zones of materials prepared by particular methods with MSRA isolates. * refers to statistically significant differences (Mann-Whitney test, *p* = *0.05).*
Fig. 3. Releasing of the active form of vancomycin from the materials prepared by impregnation in the blood plasma environment (median, interquartile range). * refers to statistically significant differences (Mann-Whitney test, *p* = *0.05).*
Fig. 4. Inhibition zones sizes for materials prepared by impregnation with different hydroxyapatite concentrations and the positive control (control discs EUCAST). * refers to statistically significant differences (mean, standard deviation, Mann-Whitney test, *p* = *0.05)* compared with the positive control.
Fig. 5. Fluorescent images of osteoblasts cultivated for 2 and 8 days on the impregnated layers and the layers before the impregnation (N0, N5, and N15).

### Preferred embodiments of the invention

Hereinafter, the examples of the embodiments of the invention are described, where the nanolayer is prepared by electrostatic spinning of collagen type I, with the addition of hydroxyapatite fraction 50 - 250 nm, and with the addition of poly(ethylene oxide), such as 5 to 11 weight %, preferably 8 weight % of the solution in phosphate buffer (0.01 M sodium dihydrogen phosphate, 0.0027M potassium chloride, and 0.137M sodium chloride, pH 7.4 at 25 °C), and subsequent addition of ethanol in the ratio of 1:1 (up to 1:2) by volume to phosphate buffer. The layer and the preparation process are designed to form the collagen layer showing the mean diameter value of 100-350 nm and incorporating homogeneously dispersed hydroxyapatite nanoparticles. The layer can be electrostatically coated directly on the surface of the metal implant.

The preparation procedure is as follows: the weighed amount of lyophilized collagen and poly(ethylene oxide) (PEO) of the molecular weight in the range of 400,000 - 900,000 is mixed with phosphate buffer. The weight ratio of poly(ethylene oxide) to collagen is 0.05 to 0.11, preferably 0.08. The prepared mixture is subjected to the temperature of 32 to 42°C, preferably about 37°C, for at least 60 hours, preferably at least 72 hours. The next step is homogenization at high rotation speed in the range of 7,000-15,000 rpm, while hydroxyapatite and ethanol is added. The prepared dispersion is electrostatically spun in electrical field of the range of 200-300 kVm⁻¹, doses in the range of 100-200 µL.min⁻¹, relative humidity below 30% and temperature below 37°C. The spinning process is preferably supplemented by electroblowing, or air flow, preferably preheated to 20-25°C, with flow velocity e.g. 15-50 L.min⁻¹. The air is directed to the area around jets mouth and it positively affects the formation of the fibres from the said solution. The layer stability after spinning is enhanced by dipping it in 95% solution of alcohol (e.g. ethanol) and water and N-(3-dimethylaminopropyl)-N-ethylcarbodiimide hydrochloride (EDC) and N-hydroxysuccinimide (NHS) at the temperature of 32 to 42 °C, preferably about 37 °C, for 18 to 30 hours, preferably about 24 hours, which leads to crosslinking. There is at least 0.625 g EDC and 0.156 g NHS, i.e. the weight ratio EDC:NHS 4:1, mixed with at least 140 mL of 95% alcohol per 1 g of collagen. After crosslinking, the layer is washed in saline solution or phosphate buffer for at least 2 x 30 min and finally washed with distilled water for at least 30 minutes. During crosslinking and washing of the layer, poly(ethylene oxide) is basically completely washed out. The layer is vacuum freeze dried. The layer prepared by this method comprises 85-100 weight % of collagen and up to 15 weight % of hydroxyapatite. Ethylene oxide sterilization or gamma ray of standard parameters can be used for sterilization of the layer. Spinning of the dispersion of collagen and hydroxyapatite has the advantage of achieving a homogenous and volume consistent layer by the combination of solution parameters and spinning parameters. Preferably, the antibiotics are also deposited. The antibiotics are deposited into the layer by impregnation in the dispersion of ethanol or water or saline solution, having the concentration of antibiotics from 10 to 50 weight % The impregnation can be performed either by immersing the implant with the deposited layer into the dispersion, or by depositing the dispersion on the layer by a brush, optionally by spraying. After the application of the dispersion, the layer is left at the room temperature until the solvent evaporates. The useful antibiotics comprise for example gentamicin, vancomycin, tobramycin, or a combination thereof, such as vancomycin and gentamicin in various weight ratios. The impregnation effectivity is shown below by the example with vancomycin.

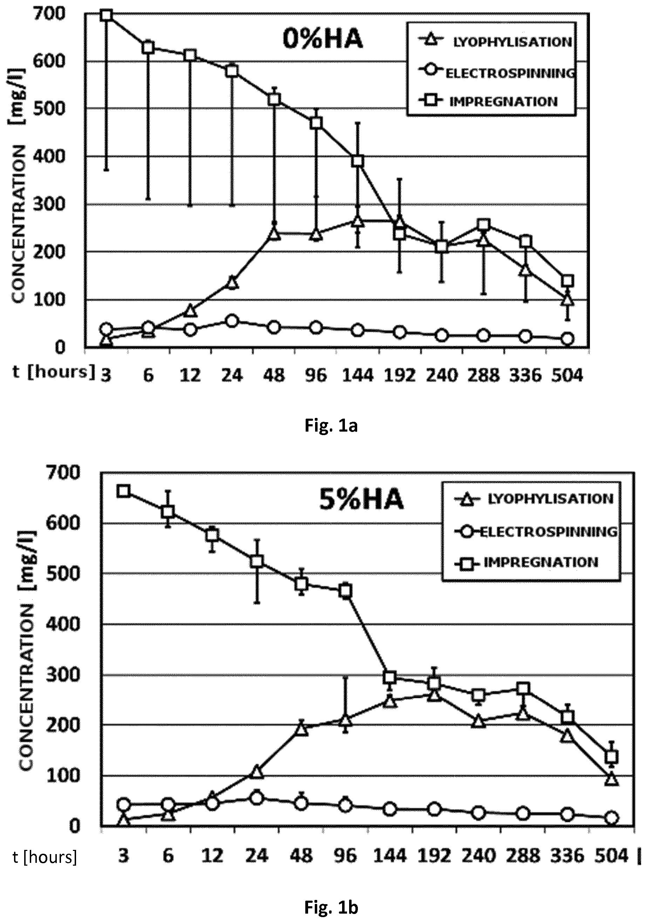

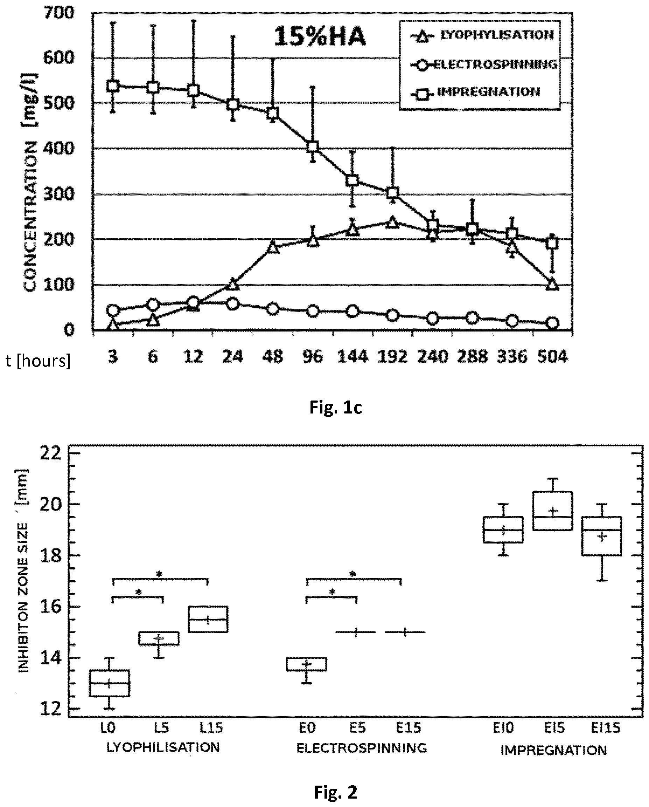

The impregnation of the layers (EI0, EI5, and EI15) was chosen as the optimal method of depositing the antibiotic in comparison with two other methods of the preparation - lyophilisation of collagen dispersion, 0, 5, and 15 weight % of hydroxyapatite and 10 weight % of vancomycin (L0, L5, and L15), electrostatic spinning of collagen dispersion, 0, 5, and 15 weight % of hydroxyapatite and 10 weight % of vancomycin (E0, E5, and E15). The influence of the particular preparation methods and structural properties of the layers on vancomycin release kinetics was determined by means of high performance liquid chromatography, and the results are shown in the Fig. 1.

The maximum concentration of vancomycin released form the material prepared by lyophilisation was achieved at day 8, namely about 250 mg.L⁻¹. The material prepared by electrostatic spinning showed the maximum value after 24 hours, namely about 60 mg.L⁻¹, wherein the impregnated material reached the maximum concentration after 3 hours, namely about 700 mg.L⁻¹. The released concentrations of vancomycin active form exceeded the minimum inhibition concentration (16 mg.L⁻¹) 17-times for the lyophilized material, 4-times for the material prepared by electrostatic spinning and 44-times for the impregnated material. The concentration of vancomycin active form is maintained above the minimum inhibition concentration for 21 days in all the materials. The results of the investigation of similar material according to the literature (Wachol-Drevek 1996 and Tu et al. 2012) show that 90% of vancomycin were released in 24 hours and the total time of releasing reached 4 days. The impregnated material shows the highest effectivity, which was proven also by the preliminary tests of antimicrobial sensitivity (Fig. 2).

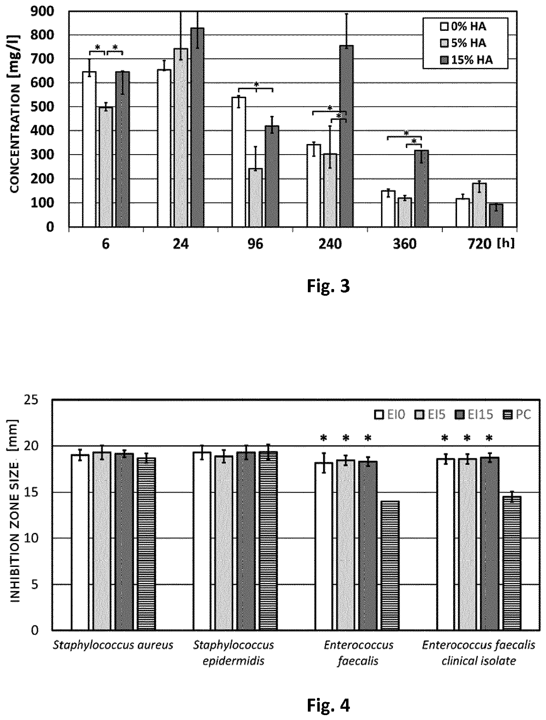

The material prepared by the impregnation was also studied for vancomycin release in blood plasma. As it is evident from Fig. 3, the material is still able to release about 62 mg.L⁻¹ of vancomycin active form even after 30 days, which exceeds the minimum inhibition concentration almost 4-times. The effect of hydroxyapatite concentration is not apparent.

This material was also tested for antimicrobial sensitivity to four bacteria stems. The sizes of the inhibition zones of particular bacteria stems are presented in Fig. 4. All the tested materials showed antibacterial activity against both the isolates of *Staphylococci* and the gentamycin resistant isolates. The inhibition zones size was comparable to the positive control (standard disc, EUCAST). The negative control (the sample without vancomycin) showed zero inhibition zone (not shown in the diagram).

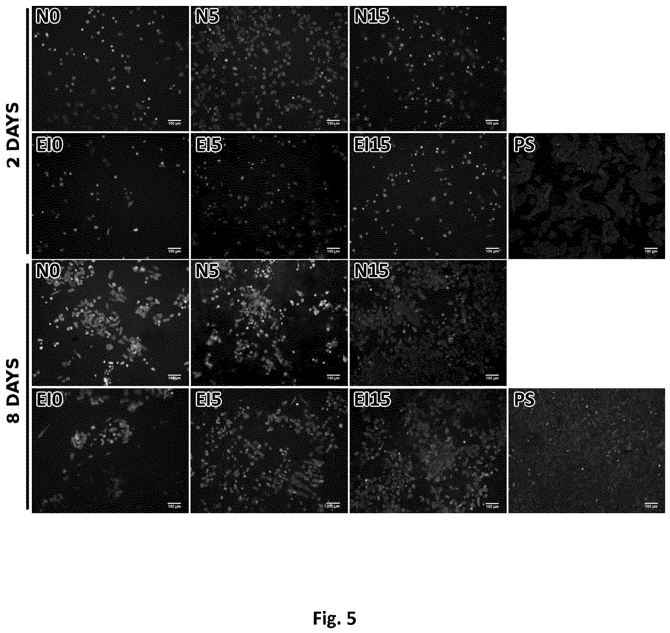

The impregnated layers were then tested for cytotoxicity and cytocompatibility *in vitro* with the use of bone cells (SAOS-2, Deutsche Sammlung von Mikroorganismen und Zellkulturen GmbH, Germany). The cells were cultured both in liquor environment and directly on the layers, and their metabolic activity was assessed (MTS test), their adhesion and proliferation was examined microscopically. The tests proved that the amounts of the released antibiotics are not toxic, the layers are cytocompatible. In Fig. 5, there are illustrative images of fluorescent stained cells after two and eight days of culture on the layers with vancomycin, without vancomycin (N0, N5, and N15), and on the positive control (PS).

### EXAMPLE 1 (not within the scope of claim 1)

The nanolayer was prepared by electrostatic spinning of collagen type I as an 8 weight % solution in phosphate buffer, followed by an addition of ethanol (1:1 vol.), to which poly(ethylene oxide) was added in the weight ratio of PEO to collagen 0.08. The collagen fibres showed the mean diameter value about 220 nm. The layer was electrostatically deposited directly onto the surface of the metal implant. The preparation process was as follows: the weighed amount of lyophilised collagen and poly(ethylene oxide) of the molecular weight 900,000 were mixed with phosphate buffer. The prepared mixture was subjected to the temperature of 37°C for 72 hours. The next step of the preparation was high speed rotation homogenization at 10,000 rpm with simultaneous adding of ethanol. The prepared dispersion was electrostatically spun in the electric field of 250 kV.m⁻¹, with doses of 130 µl.min⁻¹, relative air humidity of 25 % and the temperature of 30°C. The spinning process was accompanied by electroblowing, or preheated air flow of the temperature of 24°C and flow speed of 35 L.min⁻¹. The layer stability was enhanced after the spinning by dipping in the solution of 95 weight % of ethanol with water and EDC and NHS at the temperature of 37°C for 24 hours. Per 1 g of collagen, there is at least 0.625 g of EDC and 0.156 g of NHS mixed with 140 mL of 95% alcohol. After the crosslinking, the layer was washed with 0.1 M Na₂HPO₄ for 2 x 30 min and finally washed with distilled water for 30 min. During the crosslinking and washing of the layer, poly(ethylene oxide) was basically completely washed out. The layer was vacuum freeze-dried. The layer prepared by this method comprised 100 weight % of collagen. Ethylene oxide sterilization was used for sterilization of the layer. Then the antibiotics were deposited into the layer by impregnation in ethanol dispersion having the concentration of the antibiotic of 20 weight %. Vancomycin was used as the antibiotic. The impregnation was performed by the application of the antibiotic onto the deposited layer with a brush, so as to reach the final concentration of the antibiotic in the layer (after evaporation of the solvent) of 8 weight % based on the total weight of the layer. After the application of the dispersion the layer was kept at the room temperature until the evaporation of the solvent.

### EXAMPLE 2

The nanolayer was prepared by electrostatic spinning of collagen type I as a 10 weight % solution in phosphate buffer, followed by an addition of ethanol (1:1 vol.) to which poly(ethylene oxide) was added in the weight ratio of PEO to collagen 0.10. The collagen fibres showed the mean diameter value about 180 nm and they integrated into themselves the dispersed hydroxyapatite nanoparticles of the fraction of 170 nm. The layer was electrostatically deposited directly onto the surface of the metal implant. The preparation process was as follows: the weighed amount of the lyophilised collagen and poly(ethylene oxide) of the molecular weight of 800,000 was mixed with phosphate buffer. The prepared mixture was subjected to the temperature of 35°C for 88 hours. The next preparation step was high speed rotation homogenization at 12,000 rpm with adding of hydroxyapatite in the weight ratio to collagen 5:95, and ethanol. The prepared dispersion was electrostatically spun in the electric field of 280 kV.m⁻¹, with doses of 140 µl.min⁻¹, relative air humidity of 25% and the temperature of 27°C. The spinning process was accompanied by electroblowing, or preheated air flow of the temperature of 22°C and flow speed 40 L.min⁻¹. The layer stability was enhanced after the spinning by dipping in the solution of 95 weight % of ethanol and EDC and NHS at the temperature of 37 °C for 24 hours. Per 1 g of spun collagen, there is at least 0.800 g EDC and 0.200 g NHS mixed with 200 mL of 95% alcohol. After the crosslinking, the layer was washed with 0.1M of Na₂HPO₄ for 2 x 40 min and finally washed in distilled water for 30 min. During the crosslinking and washing the layer, poly(ethylene oxide) was basically completely washed out. The layer was vacuum freeze-dried. The layer prepared by this method comprised 95 weight % of collagen and 5 weight % of hydroxyapatite. Ethylene oxide sterilization was used for the sterilization of the layer. The antibiotics were deposited into the layer by impregnation with an aqueous dispersion having the concentration of antibiotic 30 weight %. Gentamicin was used as the antibiotic. The impregnation was performed by the application of the antibiotic dispersion onto the deposited layer with a brush so as to reach the final concentration of the antibiotic in the layer (after evaporation of the solvent) of 10 weight % based on total weight of the layer. After the application of the dispersion, the layer was kept at the room temperature until the solvent evaporation.

### EXAMPLE 3

The nanolayer was prepared by electrostatic spinning of collagen type I as an 8 weight % solution in phosphate buffer and ethanol (1:1 vol.) to which poly(ethylene oxide) was added in the weight ratio of PEO:collagen 0.08. The collagen fibres showed a mean diameter value of about 310 nm and they integrated into themselves homogenously dispersed hydroxyapatite nanoparticles of the fraction of 190 nm. The layer was electrostatically deposited directly onto the surface of the metal implant. The preparation process was as follows: the weighed amount of the lyophilised collagen and poly(ethylene oxide) of the molecular weight of 700,000 were mixed with phosphate buffer. The prepared mixture was subjected to the temperature of 40°C for 92 hours. The next preparation step was the high speed rotation homogenization at 15,000 rpm, during the homogenization hydroxyapatite in the weight ratio to collagen 15:85 and ethanol were added. The prepared dispersion was electrostatically spun in the electric field of 270 kV.m⁻¹, with dosing 150 µl.min⁻¹, relative air humidity of 26% and the temperature of 25°C. The spinning process was accompanied by electroblowing, or preheated air flow of the temperature of 24°C and flow rate 45 L.min⁻¹. The layer stability was enhanced after the spinning by dipping in the solution of 95 weight % of alcohol with water and EDC and NHS at the temperature of 37 °C for 24 hours. Per 1 g of the spun collagen, there is at least 0.920 g of EDC and 0.230 g of NHS mixed with at least 250 mL of 95% alcohol. After the crosslinking, the layer was washed with 0.1M of Na₂HPO₄ for 2 x 35 min and finally washed in distilled water for 40 min. During the crosslinking and washing of the layer, poly(ethylene oxide) was basically completely washed out. The layer was vacuum freeze-dried. The layer prepared by this method comprised the nanofibers with 85 weight % of collagen and 15 weight % of hydroxyapatite. Ethylene oxide sterilization was used for the sterilization of the layer. The antibiotics were deposited into the layer by impregnation in the dispersion of alcohol, having the concentration of the antibiotic of 30 weight %. The combination of gentamicin and vancomycin in the weight ratio 1:1 was used as the antibiotic. The impregnation was performed by spraying the dispersion. After the application of the dispersion, the layer was kept at the room temperature until the solvent evaporation. The impregnation was performed by application of the antibiotic dispersion onto the deposited layer with a brush so as to reach the final concentration of the antibiotic in the layer (after the evaporation of the solvent) of 11 weight % based on the total weight of the layer.

Although many preferred embodiments were described, it is evident that a person skilled in the art will readily find other possible alternative embodiments. Thus the scope of the invention is not limited by these preferred embodiments and it is rather defined by the appended claims.

### Industrial applicability

Orthopaedic and dental implants based on a metal or metal alloys coated with a bioactive layer of collagen type I, optionally also comprising hydroxyapatite and a deposited antibiotic, prepared according to this invention, may be used in human and veterinary medicine, especially in orthopaedics and stomatology.

## Claims

1. A method of preparation of a bioactive nanocomposite layer based on collagen nanofibers for application for orthopaedic and dental implants based on metal or metal alloys **characterized in that** collagen type I and poly(ethylene oxide), having the viscosity average molecular weight in the range of 400,000 to 900,000, the ratio of the poly(ethylene oxide) and collagen being 0.05 to 0.11, are mixed with phosphate buffer to obtain the concentration of 5 to 11 weight % of collagen in the mixture, then the resulting mixture is subjected to the temperature in the range of 32 to 42°C for at least 60 hours, then it is homogenized by mixing while simultaneously ethanol is being added, with the final volume ratio of phosphate buffer to ethanol from 1:1 to 1:2, and while hydroxyapatite in the form of nanoparticles with a size of 50-250 nm is added in the amount of 5 to 15 wt. %, based on the amount of collagen, to the solution during the homogenization, then the mixture undergoes electrostatic spinning to form the nanocomposite layer that is washed with saline solution or phosphate buffer and then with water and is dried, said nanofibers comprising at least 75 weight% of collagen type I and at most 1 weight% of poly(ethylene oxide), and having the mean diameter value of 100-350 nm.

2. The method of preparation according to claim 1 **characterized in that** the nanocomposite layer is, prior to washing, stabilized by dipping in 95 wt. % solution of alcohol in water with an addition of N-(3-dimethylaminopropyl)-N-ethylcarbodiimide hydrochloride (EDC) and N-hydroxysuccinimide (NHS), where the amount of N-(3-dimethylaminopropyl)-N-ethylcarbodiimide hydrochloride per 1 g of the spun collagen is at least 0.625 g and the amount of N-hydroxysuccinimide per 1 g of the spun collagen is at least 156 mg per 1 g of the spun collagen, and where the amount of the 95 wt. % solution of alcohol in water is at least 140 ml per 1 g of the spun collagen.

3. The method of preparation according to any of claims 1 to 2 **characterized in that** after drying, the layer is impregnated with an antibiotic or a mixture of antibiotics selected from the group of vancomycin, gentamicin, and tobramycin.

4. The method of preparation according to claim 3 **characterized in that** the impregnation is performed by dipping the layer into the dispersion or depositing the dispersion onto the layer or spraying the dispersion onto the layer, where the dispersion is a dispersion of antibiotics in alcohol, water, or in a mixture of alcohol and water.

## Patentansprüche

1. Ein Verfahren zur Herstellung einer bioaktiven Nanokompositschicht auf der Basis von Kollagennanofasern, die zum Auftragen auf orthopädische sowie zahnmedizinische Implantate auf der Basis von Metallen und metallischen Legierungen, **dadurch gekennzeichnet, dass** Kollagen mit der Typenbezeichnung I und ein Polyethylenoxid mit einem im Bereich von 400000 bis 900000 liegenden Viskositätsmittel Molekulargewicht bei einem im Bereich von 0,05 bis 0,11 liegenden Verhältnis zwischen Kollagen und Polyethylenoxid, mit Phosphatpuffer vermischt werden, um eine im Bereich von 5 bis 11 Gew.-% liegende Konzentration von Kollagen in dem Gemisch zu erreichen, worauf das derart erhaltene Gemisch der mindestens 60 Stunden lang dauernden Einwirkung der im Bereich von 32 bis 42 °C liegenden Temperatur ausgesetzt wird, in Anschluss daran wird das Gemisch durch Umrührung unter gleichzeitiger Zugabe von Ethanol in dem endgültigen Volumenverhältnis zwischen Phosphatpuffer und Ethanol von 1:1 bis 1:2 homogenisiert, wobei während der Homogenisierung der Lösung gleichzeitig Hydroxyapatit in der Form von Nanopartikeln in dem Grössenbereich von 50 bis 250 nm in der Menge von 5 bis 15 Gew.-%, die auf die Menge von Kollagen bezogen ist, zugegeben wird, worauf das Gemisch unter Bildung einer Nanokompositschicht elektrostatisch versponnen wird, welche Schicht anschliessend mit einer physiologischen Lösung oder mit Phosphatpuffer und daraufhin mir Wasser durchgewaschen und getrocknet wird, wobei die Nanofasern wenigstens 75 Gew.-% von Kollagen mit der Typenbezeichnung I und höchstens 1 Gew.-% von Polyethylenoxid enthalten und wobei die Nanofaser einen Mittelwert der Durchmesser im Bereich von 100 bis 350 nm aufweist.

2. Das Verfahren zur Herstellung nach Anspruch 1, **dadurch gekennzeichnet, dass** die Nanokompositschicht vor dem Durchwaschen durch Einweichen in der 95 Gew.-% Lösung eines Alkohols in Wasser mit einem Zusatz von N-(3-Dimethylaminopropyl)-N-ethylcarbodiimid-Hydrochlorid (EDC) und N-Hydroxysuccinimid (NHS) stabilisiert wird, wobei die auf 1 g vom versponnenen Kollagen bezogene Menge von N-(3-Dimethylaminopropyl)-N-ethylcarbodiimid-Hydrochlorid mindestens 0,625 g beträgt und die auf 1 g vom versponnenen Kollagen bezogene Menge von N-Hydroxysuccinimid mindestens 156 mg beträgt und wobei die auf 1 g vom versponnenen Kollagen bezogene Menge der 95 Gew.%-igen Lösung eines Alkohols in Wasser mindestens 140 ml beträgt.

3. Das Verfahren zur Herstellung nach Anspruch 1 oder 2, **dadurch gekennzeichnet, dass** die Schicht nach Trocknen mit einem Antibiotikum oder einem Gemisch von aus der Vankomycin, Gentamicin und Tobramycin umfassenden Gruppe ausgewählten Antibiotika imprägniert wird.

4. Das Verfahren zur Herstellung nach Anspruch 3, **dadurch gekennzeichnet, dass** die Imprägnierung durch Einweichen der Schicht in eine Dispersion oder durch Auftragen oder Zerstäuben einer Dispersion auf die Schicht erfolgt, wobei die Dispersion eine Dispersion von Antibiotika in einem Alkohol, im Wasser oder in einem Gemisch von einem Alkohol und Wasser ist.

## Revendications

1. Un procédé de préparation d'une couche nanocomposite bioactive à base de nanofibres de collagène pour des applications pour des implants orthopédiques et dentaires à base de métal ou d'alliages métalliques, **caractérisé en ce que** le collagène de type I et le poly(éthylène oxyde), ayant la masse molaire moyenne viscosimétrique dans la plage de 400000 à 900000, le rapport du poly(éthylène oxyde) au collagène étant de 0,05 à 0,11, sont mélangés avec le tampon phosphate saline pour obtenir la concentration de 5 à 11 % en poids de collagène dans le mélange et ensuite le mélange qui en résulte est soumis à la temperature dans la plage de 32 à 42 °C pendant au moins 60 heures, puis il est homogéneisé par malaxage tout en ajoutant de l'éthanol, le rapport volumique final du tampon phosphate saline à l'éthanol étant de 1:1 à 1:2 et en même temps l'hydroxyapatite sous la forme de nanoparticules, ayantes une dimension de 50 à 250 nm, est ajouté à la solution pendant la homogénéisation dans la quantité de 5 à 15 % en poids, par rapport à la quantité de collagène, puis le mélange subit un filage électrostatique pour former la couche nanocomposite qui est lavée avec de la solution saline ou le tampon phosphate saline et puis avec de l'eau et ensuite est séchée, lesdits nanofibres comprenants au moins 75 % en poids de collagène de type I et au plus 1 % en poids de poly(éthylène oxyde) et ayants la valeur moyenne de diamètre de 100 à 350 nm.

2. Le procédé de préparation selon la revendication 1 **caractérisé en ce que** la couche nanocomposite est, avant le lavage, stabilisée par immersion dans une solution du 95 % en poids d'alcool dans l'eau avec un ajout de N-(3-diméthylaminopropyl)-N-éthylcarbodiimide chlorhydrate (EDC) et de N-hydroxysuccinimide (NHS), où la quantité de N-(3-diméthylaminopropyl)-N-éthylcarbodiimide chlorhydrate pour 1 g du collagène filé est au moins 0,625 g et la quantité de N-hydroxasuccinimide pour 1 g du collagène filé est au moins 156 mg et où la quantité de la solution du 95 % en poids d'alcool dans l'eau est au moins 140 ml pour 1 g du collagène filé.

3. Le procédé de préparation selon l'une quelconque des revendications 1 à 2 **caractérisé en ce qu'**après le séchage, la couche est imprégnée avec un antibiotique ou avec un mélange d'antibiotiques sélectionnés dans le groupe de vancomycin, gentamicin et tobramycin.

4. Le procédé de préparation selon la revendication 3 **caractérisé en ce que** l'imprégnation est effectué par immersion de la couche dans la dispersion ou par dépôt de la dispersion sur la couche ou par pulvérisation de la dispersion sur la couche, où la dispersion est une dispersion d'antibiothiques dans l'alcool, dans l'eau ou dans un mélange d'alcool et d'eau.
